# EUROPEAN PATENT SPECIFICATION

(11) **EP 0 943 015 B1**
(45) Date of publication and mention of the grant of the patent: **18.09.2002**
(21) Application number: 97948294.0
(22) Date of filing: 13.11.1997
(51) Int. Cl.: C12Q 1/68, G01N 1/30, C12M 1/24, C12M 1/10, C12M 1/04

(54) **METHOD AND APPARATUS FOR PREPARING SAMPLE CARTRIDGES FOR A PARTICLE ACCELERATION DEVICE**
VERFAHREN UND VORRICHTUNG ZUR HERSTELLUNG VON PROBENRÖHRCHEN FÜR EINE PARTIKELBESCHLEUNIGUNGSEINRICHTUNG
PROCEDE DE PREPARATION DE CARTOUCHES D'ECHANTILLONS POUR UN ACCELERATEUR DE PARTICULES

(30) Priority: 13.11.1996 US 747870
(43) Date of publication of application: 22.09.1999
(73) Proprietor: Powderject Vaccines, Inc., Madison, Wisconsin 53711 (US)
(72) Inventor: McCABE, Dennis, E., Middleton, WI 53562 (US)
(74) Representative: Price, Nigel John King
(86) International application number: PCT/US97/20817
(87) International publication number: WO 98/021364

(56) References cited:
- EP-A- 0 362 863
- WO-A-96/12513
- SANFORD, J.C. et al. Delivery of substances into cells and tissues using a particle bombardment process. Parti- culate science and technoloy. 1987, Vol. 5, No. 1, pages 27-37, XP002900192

## Description

### Technical Field

The present invention relates to the field of particle delivery. More particularly, the invention relates to a particle-mediated delivery method and apparatus for delivering materials into a target cell.

### Background of the Invention

In the past decade, particle-mediated acceleration of biological and pharmaceutical materials, particularly genetic material, into living cells and tissue, has emerged as an important tool for use in the fields of plant and animal biotechnology. Transient expression and germ line integration of introduced DNA has been demonstrated in microorganisms, plants, and animals.

As the fundamentals of the technology have been elucidated, attention has increasingly shifted toward development of devices that enable one to perform particle-mediated delivery of biological materials sequentially, and in rapid succession. Such a device would be particularly advantageous for use in mass immunization of humans or domesticated animals with various vaccine compositions.

To that end, an instrument has been developed for accelerating particles coated with biological substances using compressed gas as the motive force. The biological materials are deposited upon the surface of small, dense particles of a carrier material, such as gold or platinum, which may be spherically shaped. The coated carrier particles are then coated onto the interior curved surface of a rigid tube or cartridge. The coated tube or cartridge is loaded into the instrument and aligned with a barrel. Upon release from a suitable source, compressed gas is passed through the coated tube and the barrel, which picks up the carrier particles and accelerates the same toward a target surface.

Thus it is advantageous to uniformly coat the interior of the tube with particles that carry the biological sample and to produce the same uniform coating on numerous tubes.

### Summary of the Invention

It is a general object of the present invention to provide a method and an apparatus for forming a large number of particle cartridges for use in a gas driven particle acceleration instrument. A further object of the invention is to provide such a method and an apparatus which uniformly coats the cartridges with the particles.

In one embodiment, an apparatus is provided for depositing particles within a length of tubing. The apparatus comprises a tubing roller having an elongate tubing bore formed therein, wherein said bore has first and second ends and is sized for removable insertion of a length of tubing therein. A means for rotating the tubing roller about the major axis of the tubing bore is also provided, and a gas delivery means, which comprises a chamber with an inlet for introducing gas from an associated source into the chamber, and an aperture through which a portion of the tubing roller extends. The aperture provides fluid communication between the second end of the tubing bore and the chamber. A support means is arranged adjacent to the first end of the tubing bore, wherein the support means provides for the sealable engagement between an end of a length of tubing inserted into the tubing bore and an associated source of particles to be deposited within the length of tubing.

In another embodiment, a method is provided for depositing particles in a length of tubing having a longitudinal axis and a curved interior surface. The method comprising the following steps:
(a) preparing a uniformly dispersed suspension of particles coated with a biological substance in an evaporable liquid;
(b) rotating the tubing about its longitudinal axis at a first speed;
(c) introducing the particle suspension into the tubing while rotating said tubing at the first speed;
(d) rotating the tubing to centrifugally separate the particles from the evaporable liquid and distribute the particles on the interior surface of the tubing; and
(e) passing a gas through the tubing as the tubing rotates to dry the particles distributed on the interior surface.

It is an advantage of the present invention that a large number of substantially identical sample cartridges can be prepared in a single effort. The present centrifugal method produces tubes that are very uniform, as compared to previous techniques, and lends itself to automation of the process.

These and other objects, features and advantages of the present invention will become apparent from the following specification, read in light of the accompanying drawings.

### Brief Description of the Drawings

Figure 1 is a cut-away view of an apparatus constructed according to the present invention.
Figure 2 is a cross-sectional view taken along line 2-2 in Figure 1.
Figure 3 is a flowchart depicting the sequence of steps in a process for depositing coated particles within a tubular sample cartridge.

### Detailed Description of the Preferred Embodiment

Before describing the present invention in detail, it is to be understood that this invention is not limited to a particular particle delivery device, or to particular carrier particles as such may, of course, vary. It is also understood that different embodiments of the disclosed sample method and apparatus may be tailored to the specific needs in the art. It is also to be understood that the terminology used herein is for the purpose of describing particular embodiments of the invention only, and is not intended to be limiting.

All publications, patents and patent applications cited herein, whether supra or infra, are hereby incorporated by reference in their entirety.

It must be noted that, as used in this specification and the appended claims, the singular forms "a", "an", and "the" include plural referents unless the content clearly dictates otherwise. Thus, for example, reference to "a particle" includes reference to mixtures of two or more particles, reference to "a therapeutic agent" encompasses one or more such agents, reference to "a bearing mount" includes one or more such mounts, and the like.

### A. Definitions

Unless defined otherwise, all technical and scientific terms used herein have the same meaning as commonly understood by one of ordinary skill in the art to which the invention pertains. The following terms are intended to be defined as indicated below.

As used herein, the term "therapeutic agent" intends any compound or composition of matter which, when administered to an organism (human or nonhuman animal) induces a desired pharmacologic, immunogenic, and/or physiologic effect by local, regional, and/or systemic action. The term therefore encompasses those compounds or chemicals traditionally regarded as drugs, vaccines, and biopharmaceuticals including molecules such as proteins, peptides, hormones, nucleic acids, gene constructs and the like. Such therapeutic agents may be used prophylactically to prevent disorders and/or for the treatment of on-going disorders.

More particularly, the term "therapeutic agent" includes compounds or compositions for use in all of the major therapeutic areas including, but not limited to, anti-infectives such as antibiotics and antiviral agents; analgesics and analgesic combinations; local and general anesthetics; anorexics; antiarthritics; antiasthmatic agents; anticonvulsants; antidepressants; antihistamines; anti-inflammatory agents; antinauseants; antineoplastics; antipruritics; antipsychotics; antipyretics; antispasmodics; cardiovascular preparations (including calcium channel blockers, beta-blockers, beta-agonists and antiarrythmics) ; antihypertensives; diuretics; vasodilators; central nervous system stimulants; cough and cold preparations; decongestants; diagnostics; hormones; bone growth stimulants and bone resorption inhibitors; immunosuppressives; muscle relaxants; psychostimulants; sedatives; tranquilizers; proteins, peptides and fragments thereof (whether naturally occurring, chemically synthesized or recombinantly produced); and nucleic acid molecules (polymeric forms of two or more nucleotides, either ribonucleotides (RNA) or deoxyribonucleotides (DNA) including both double- and single-stranded molecules, gene constructs, expression vectors, antisense molecules and the like).

Particles of a therapeutic agent, alone or in combination with other drugs or agents, are typically prepared as pharmaceutical compositions which can contain one or more added materials such as vehicles, and/or excipients. "Vehicles" and "excipients" generally refer to substantially inert materials which are nontoxic and do not interact with other components of the composition in a deleterious manner. These materials can be used to increase the amount of solids in particulate pharmaceutical compositions. Examples of suitable carriers include water, silicone, gelatin, waxes, and like materials. Examples of normally employed "excipients," include pharmaceutical grades of dextrose, sucrose, lactose, trehalose, mannitol, sorbitol, inositol, dextran, starch, cellulose, sodium or calcium phosphates, calcium sulfate, citric acid, tartaric acid, glycine, high molecular weight polyethylene glycols (PEG), and combinations thereof. In addition, it may be desirable to include a charged lipid and/or detergent in the pharmaceutical compositions. Such materials can be used as stabilizers, anti-oxidants, or used to reduce the possibility of local irritation at the site of administration. Suitable charged lipids include, without limitation, phosphatidylcholines (lecithin), and the like. Detergents will typically be a nonionic, anionic, cationic or amphoteric surfactant. Examples of suitable surfactants include, for example, Tergitol® and Triton® surfactants (Union Carbide Chemicals and Plastics, Danbury, CT), polyoxyethylenesorbitans, e.g., TWEEN® surfactants (Atlas Chemical Industries, Wilmington, DE), polyoxyethylene ethers, e.g., Brij, pharmaceutically acceptable fatty acid esters, e.g., lauryl sulfate and salts thereof (SDS), and like materials.

### B. The Deposition Apparatus

The present invention provides a reproducible method for mass producing sample cartridges for use in a gas-driven particle acceleration instrument. In particular, small dense carrier particles are reversibly coated onto a concave inner surface of a sample cartridge. The carrier particles are themselves reversibly coated with a therapeutic agent, for example, a biological substance such as genetic material or a protein. During particle acceleration and delivery, a gas stream passing over the carrier particles releases the same from the inner surface of the sample cartridge, and carries the particles to a target cell, tissue, or organism.

For repeatability of delivery, it is important that the number of particles delivered from each sample cartridge be ascertainable and relatively constant, at least within a statistically acceptable range, for example, within about ± 10% of an experimentally determined mean number. It is also important that particle distribution among the sample cartridges be kept substantially constant, thus maximizing sample-to-sample reproducibility.

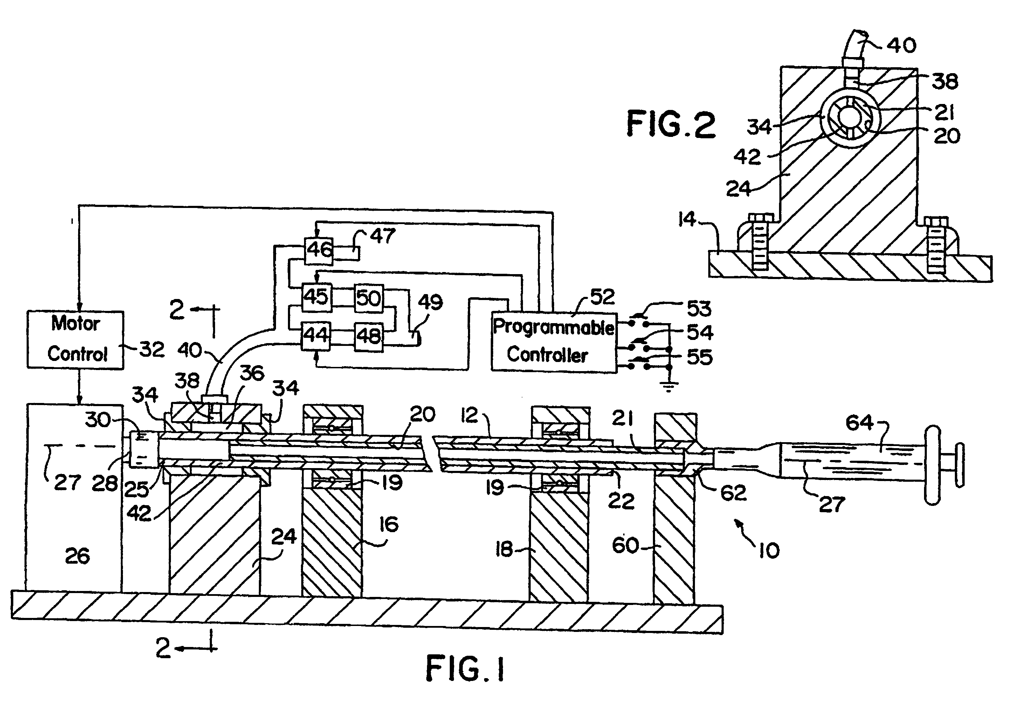

Referring now to Figure 1, a suitable apparatus **10** is shown which can be used in the deposition procedure described herein. The apparatus **10** comprises a tubing roller **12** which is rotatably mounted to a base **14** in a generally horizontal orientation by two or more mounts, depicted at **16** and **18**. The base **14** can be of any size and shape, and should be at least as long as the length of tubing to be coated in the practice of the present invention. The base **14** can include leveling means and a spirit level to facilitate the horizontal positioning of the tubing roller. The mounts **16** and **18** are attached to, or part of, the base **14**, and comprise bearing mounts, indicated at **19**, which engage and retain the tubing roller **12** in its horizontal position in the apparatus. The bearing mounts **19** can be of any suitable type, e.g., roller, ball or needle type, so long as they allow precise rotation of the tubing roller **12** about its major axis with minimal friction. The mounts **16** and **18** are preferably rotatably engaged with the tubing roller **12** at opposing ends thereof. Depending upon the length of the tubing roller **12**, additional mounts may be provided between the terminally positioned mounts **16** and **18** as needed to prevent excessive vibration in the tubing roller at the relatively high rotational speed used during the coating process.

The tubing roller **12** can be formed of any substantially rigid, durable material such as metal, plastic, wood or the like. In the embodiment depicted in Figure 1, the tubing roller **12** is cylindrical. In addition, the tubing roller **12** has sufficient length along the axis of rotation to receive and secure substantially the entire length of a piece of tubing which is to be coated.

The mounted tubing roller **12** has an axis of rotation which is coaxial with the major axis thereof, and an elongate tubing bore **20** which is coaxial with the axis of rotation. The tubing bore **20** is positioned so that a length of tubing **21** received therein shares the same axis of rotation as the tubing roller **12** and extends into the tubing roller through an opening **22** at a first end of the tubing bore. The tubing bore **20** is sized in length, width, and depth, to accommodate a variety of tubing types, and preferably is generally cylindrical. To facilitate insertion of the tubing **21** into the tubing bore **20**, the opening **22** of the tubing bore can be wider than the bore **20** itself and preferably flares outward from the bore **20.** A second opening **25** in the tubing bore **20** is capped. The tubing bore **20** extends along the rotational axis **27** of the apparatus **10**.

The capped second end of the tubing bore passes through a gas delivery mount **24** and engages a means **26** for rotating the tubing roller **12** about the axis of rotation (i.e., about the major axis of the elongate tubing bore). The rotator means **26** can be powered in any way, for example, using electrical or mechanical energy to effect the direct or indirect rotation of the tubing roller **12**. However, the rotator must provide sufficient power to rotate the tubing roller **12** about the axis of rotation at various constant rates between about 50 and 6000 revolutions per minute (RPM) for at least about two minutes. The rotator means **26** can be connected to any portion of the tubing roller **12**, so long as the axial rotation of the roller is not unduly constrained. In one configuration, a shaft **28** of the rotator means **26** is directly attached via fitting **30** to the capped second end **25** of the tubing bore. A suitable rotator means **26** that can attach directly to the shaft **28** is an electrically actuated gear motor, such as a Barnant Mixer Series **20** motor, which can be remotely controlled using an associated variable speed motor control, indicated at **32**. The rotator means **26** need not be attached to the base **14**, but can be attached thereto to provide for increased stability during operation of the apparatus.

As described above, a portion of the tubing roller **12** passes through a gas delivery mount **24** as shown in Figures 1 and 2. The gas delivery mount provides a gas delivery means for introducing gas into the tubing bore **20**. More particularly, the tubing roller **12** extends through an aperture in the gas delivery mount **24** and is sealably supported within the aperture by a pair of bushings **34** which are formed of a durable, low friction material. The bushings **34** seal off the opening of the aperture, and thus help define a chamber **36** within the gas delivery mount. Alternatively, sealed bearings may be utilized in place of bushings **34**, as long as the sealed bearings can operate at the rotational speeds used in the method of the present invention. An inlet passage **38** is provided in the gas delivery mount **24**. The inlet passage **38** allows for introduction of a flow of gas from an associated source into the chamber **36**, for example via gas conduit **40**. A transverse aperture **42** extends through the tubing roller **12** to provide fluid communication between the chamber **36** and the tubing bore **20**, thereby allowing gas from the chamber to enter the tubing bore. The chamber **36** extends completely around the tubing roller **12** so that communication between the gas conduit **40** and the chamber **36** can remain constant while the tubing roller is spun about axis **27** by the rotator means **26**. Thus, the gas delivery mount **24** enables continuous, uninterrupted delivery of a drying gas from an associated external source of gas into the second end of the bore **20** at a suitably controlled flow rate.

The gas conduit **40** can be connected to one or more gas valves in order to provide for controlled delivery of gas into the apparatus **10.** In the apparatus of Figure 1, three electrically operated solenoid valves **44, 45** and **46,** are connected to the gas conduit **40** to provide for variable rates of gas delivery into the apparatus. The inlet side of the first valve **44** is coupled to a first flow regulator **48** which allows gas to flow therethrough at a first, fixed rate, for example between about 2.5 - 3.5 ml/minute. Similarly the inlet side of the second valve **45** is coupled to a second flow regulator **50** which allows gas to flow therethrough at a second fixed rate, for example between about 500 - 800 ml/minute. The two flow regulators are connected via a supply hose **49** to a source of a compressed drying gas, such as air or nitrogen. As can be seen, the first valve/regulator combination provides a first gas delivery path into the chamber **36**, and the second valve/regulator combination provides a second gas delivery path into the chamber. The third valve **46** is used as an outlet for the chamber **36**, and connects the gas conduit **40** to an atmospheric exhaust port **47**.

Operation of the components of the deposition apparatus **10** may be controlled manually, but preferably is governed by a commercially available programmable controller **52**. The programmable controller **52** has outputs connected to the speed control device **32** and the three solenoid valves **44-46**. Inputs of the programmable controller **52** can be connected to three push button switches **53**, **54** and **55**, by which buttons a technician designates operating modes of load, spin and stop, respectively. As will be appreciated by the skilled artisan upon reading the instant specification, a microprocessor unit can be used to direct operation of the programmable controller, allowing for fully automated operation of the deposition apparatus **10**. For example, an appropriate set of spin cycle times, rotational rates, and drying gas flow rates, can be entered into the microprocessor, which then controls operation of the controller **52** over an entire deposition procedure. Alternatively, the microprocessor allows for semiautomatic operation of the deposition apparatus **10**, such as where one or several cycles of the deposition procedure are under the control of the microprocessor, while parameters of other operations are controlled manually.

### C. Particle Preparation

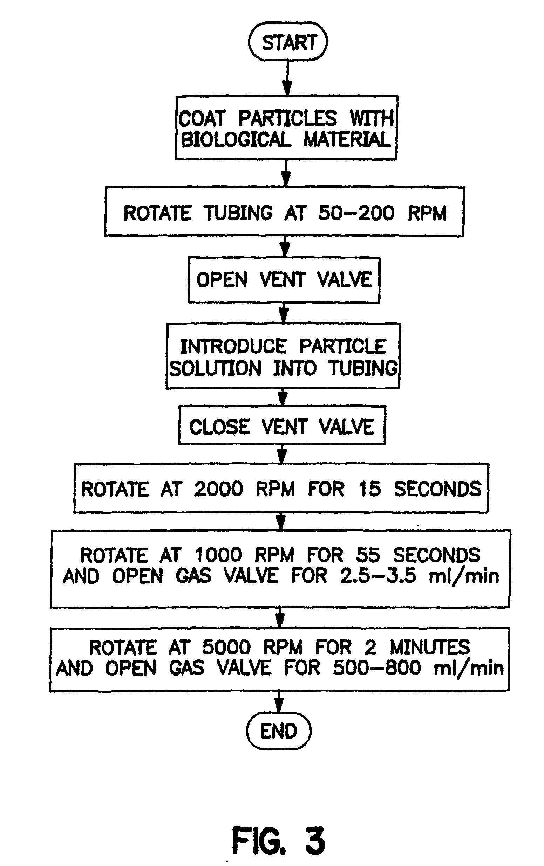

The process by which carrier particles (coated with a therapeutic agent) are deposited on the inner surface of a length of tubing is depicted in the flowchart of Figure 3. The first step in the process involves coating the carrier particles with the therapeutic agent.

Therapeutic agents (or pharmaceutical preparations derived therefrom) can be coated onto carrier particles using a variety of techniques known in the art. Dense materials are preferred in order to provide particles that can be readily accelerated toward a target over a short distance, wherein the coated particles are still sufficiently small in size relative to the cells into which they are to be delivered. It has been found that carrier particles having an average diameter of a few microns can readily enter living cells without unduly injuring such cells.

Methods for coating the small, dense particles with a biological substance are also known. Any such method can be used to prepare the coated particles, however a preferred method for coating DNA onto gold particles is described herein. One of ordinary skill in the art will appreciate from the following description the importance of determining, within acceptable tolerance limits, the amount of biological substance per particle and the number of particles per sample cartridge. The acceptable tolerance levels should be about ±30%, preferably about ±20%, and even more preferably about ±10% of the desired amount.

Gold particles are preferred for coating with DNA. References herein to "beads" or "particles" are intended to include, without limitation, both spherical and amorphous particles of appropriate size and density. DNA is a preferred biological substance for coating onto particles. However, other substances including, but not limited to, RNA and proteinaceous materials can also be coated onto particles using the following techniques. In this regard, conditions for depositing other biological substances or for using non-gold particles can vary from the method stated in ways that are understood in the art.

Continuing with the particle preparation method, a desired amount of gold particles is placed in a centrifuge tube. The amount of gold used can be roughly determined by multiplying the desired number of particles per delivery by the number of sample cartridges being prepared, e.g., the number of cartridges produced from one piece of tubing **21**. A suitable amount of particles per delivery is typically on the order of about 0.25 to 0.50 mg of gold particles per delivery, although acceptable amounts can be higher or lower. By routine experimentation, one can ascertain limits on particle delivery amounts below which the transfer is acceptably high (by any ascertainable measure, such as gene expression level or biological response to treatment) while the trauma to target tissues is minimal. Minimal trauma in an animal target tissue is evidenced by only a slight reddening of the target area.

One representative method for preparing DNA-coated particles is as follows. A small volume (100 to 300 ml) of 0.1M spermidine is added to the centrifuge tube and a suspension of nonagreggated particles is formed by sonicating the tube contents for a sufficient length of time, generally for a few seconds.

Next, an appropriate volume of DNA, suspended in a buffer that does not affect its integrity or stability, is added to the particle/spermidine suspension to achieve an acceptable DNA loading rate. The DNA, spermidine, and gold particles are mixed by vortexing. The DNA loading rate is the average density of DNA per particle, expressed for a bulk population (e.g., µg DNA per mg of particles). Preferred effective DNA loading rates on gold particles range from about 0.1 to 5.0 µg DNA per mg gold particles. Exceeding 10.0 µg DNA per mg gold is not preferred as it can lead to clumping of the gold particles. However, as little as 0.001 µg DNA per mg of gold is adequate to achieve significant expression from some expression vectors.

In order to obtain the most uniform coating results, the volume of DNA should not exceed the volume of spermidine, but smaller volumes may be used. Accordingly, it may be necessary to adjust either the concentration of DNA or the volume of spermidine added initially to the gold particles.

Calcium chloride (CaCl₂) is then added to the mixture during gentle vortexing. A sufficient amount of the CaCl₂ is added to result in precipitation of DNA-coated gold particles. If 2.5M CaCl₂ is added, a suitable volume is equal to the volume of spermidine added earlier. The mixture is allowed to precipitate at room temperature for at least five or ten minutes. At DNA loading rates of 1.0 µg DNA per mg gold particles, or higher, precipitation should be apparent immediately after the CaCl₂ is added.

After precipitation, the tube is centrifuged briefly (10-15 seconds) to pellet the coated gold particles. The supernatant is removed and discarded and the pellet is washed several times with a suitable solvent (e.g., ethanol) until virtually all of the water has been removed from the coated particle preparation. Between each solvent wash, the preparation is spun and the supernatant discarded. The coated particles of the final pellet, containing known amounts of both DNA and gold, are resuspended in an evaporable liquid, preferably 100% ethanol, optionally containing an appropriate amount of an additive that provides a slight, temporary adhesive effect sufficient for joining the coated particles to the sample cartridge. One such suitable adhesive is polyvinyl pyrrolidone (PVP). The amount of adhesive required in the evaporable liquid depends upon the gas pressure which the sample cartridges will be exposed to during subsequent particle acceleration, and also upon the type of tubing used. For delivery operations at gas pressures ranging from about 100 to 150 psi, no adhesive is required. For operation at about 150 to 300 psi, PVP at 0.001 to 0.01 mg per ml of the particle preparation is appropriate. PVP at 0.01 to 0.05 mg per ml is suitable for operations at pressures ranging from about 300 to 500 psi or higher. At operation pressures of about 500 to 800 psi, 0.3 mg per ml PVP will provide a suitable adhesive effect.

Some care should be taken in determining the total volume in which to resuspend the coated particles. The volume depends upon the desired amount of biological substance per delivery, the actual DNA loading rate, the desired particle density in the final sample cartridge, and the internal volume per length of tubing. One of ordinary skill will also recognize that the preferred amount of DNA per delivery, and the amount of particles per delivery, will vary with the nature of the target, the density at which the particles are coated, and the desired outcome of the transfer (e.g. transient expression or stable integration). Therefore, each of the stated variables, including the concentration at which the particles are loaded into the tubing, should be adjusted accordingly.

After settling upon a desired particle loading rate, particle density, and volume capacity per unit length of tubing, one can readily determine the total volume of the evaporable liquid in which to resuspend the coated particles. A suitable sample cartridge length has been found to be about a 12.7 mm length of tubing having an internal capacity of between about 0.6 and 2.0 ml per 17.78 cm length. For tubing with this particular internal capacity, a simple calculation demonstrates that if 0.5 mg of gold is desired in a 12.7 mm sample cartridge, the particles are prepared at a concentration of 7.0 mg gold per ml. Likewise, for a 0.25 mg sample in a 12.7 mm cartridge, a 3.5 mg per ml concentration is appropriate. Concentrations that achieve other particle densities are calculated in the same way.

To achieve complete transfer of the coated particles into the evaporable liquid, it is recommended that the pellet be transferred to the storage tube in several partial transfer steps. For example, the coated particles can be resuspended in a small volume (500 ml) of the liquid, vortexed, briefly sonicated (2-3 seconds), and then transferred to a clean tube. It is recommended that the tube be formed of a material to which the biological substances do not stick, such as a polypropylene culture tube. These small volume transfers can be repeated until all of the coated particles have been transferred to the tube. If desired, the tubes containing suspended coated particles can be sealed with Parafilm and stored for several months at -20°C. When the coated particles have been completely transferred, preparation of the sample cartridges can begin. Previously stored tubes should be warmed to room temperature before unsealing for use in the following tube coating method.

### Cartridge Loading Process

To prepare sample cartridges, a length of suitable tubing having a concave arcuate inner surface is filled with a uniform suspension of the coated particles dispersed within the evaporable liquid. It is preferred that the tubing is transparent or translucent so that particles coated onto the inner concave surface can be visually observed. All tubing used should be inert to reaction with the selected drying gas (preferably nitrogen) and should be sufficiently durable to retain mechanical stability throughout the particle delivery process. Tefzel® tubing (1/8" outer diameter x 3/32" inner diameter) has been found to be a suitable tubing substrate for use in the practice of the invention. A 17.78 cm length of this particular size of tubing has an internal capacity of about 0.8 ml.

Referring to again to Figure 1, when the deposition apparatus is turned off, a length of tubing **21** can be inserted through opening **22** into the tubing bore **20** such that the tubing closely engages the surface of the bore and the two components will rotate together. A portion of the tubing **21** is left projecting from the opening **22** of the bore **20**. The amount of tubing that extends from the opening is selected such that the tubing will not be inserted too far into the tubing roller **12** where it could block gas flow into the chamber **36**. A removable support **60** is then secured to the base **14** adjacent to the exposed end of the tubing **21**. The support **60** comprises a slip bearing **62** which receives the exposed end of the tubing. The slip bearing **62** engages the outer surface of the tubing **21** in a manner that provides a fluid-tight seal between the end of the tubing and the support **60**, while allowing the tubing to rotate within the slip bearing which remains stationary within the support.

A suspension of coated particles, prepared by a method such as that described above, is vortexed and sonicated to achieve a uniform distribution. A charge of the coated particle suspension is then drawn into a suitable delivery means, such as the barrel syringe **64** depicted in Figure 1. As will be appreciated upon reading the instant specification, any suitable source of particles can be used, however, the barrel syringe provides for convenient measured delivery of a volume of the particle suspension. The syringe **64** has an resilient coupling that is adapted to fit onto an exposed end of the slip bearing **62** as shown in Figure 1. In this manner, the support **60** provides for the sealable engagement between the exposed end of the tubing **21** and the syringe **64** for delivery of the particles which are to be deposited within the length of tubing.

With the filled syringe **64** attached to the slip bearing **62,** the technician presses the "load" switch **53**. Actuation of this switch causes the programmable controller **52** to open a vent valve **50**, and directs the speed control means **32** to activate the rotator means **26** which starts to turn the tubing roller **12** and the tubing **21** at a first speed, for example, 50 to 200 rpm. The plunger of the syringe **64** then is pushed by the technician to force the coated particles from the syringe into the tubing **21**. By rotating the tubing upon delivery of the particle suspension from the syringe, the particles are prevented from setting to the bottom of the horizontally arranged tubing **21**. In addition, venting the opposite end of the tubing via vent valve **50** prevents pressure build-up in the tube during introduction of the coated particles.

After the coated particles have been transferred to the tubing **21**, the syringe **64** is removed from the slip bearing **62**. Next, the technician presses the "spin" switch **54**, which causes the programmable controller **52** to direct closure of the vent valve **50** and the speed control **32** to increase the rotational speed of the tubing roller to a second speed, for example about 1700 to 2300 rpm, with 2000 rpm being preferred. The tubing spins at this second rate for at least about 15 seconds which centrifugally forces the particles out of suspension and against the concave arcuate inner surface of the tubing **21**, forming a uniform layer thereon.

After the layer forms, the settled particles are dried by first expelling the supernatant from the tubing. This is readily achieved by introducing a drying gas, such as air or nitrogen gas, into one end of the tubing. At this point, the programmable controller **52** automatically decreases the rotation speed of the tubing to between about 700 to 1300 rpm, with 1000 rpm preferred. At the same time, the first gas valve **44** is opened by the controller **52**, which causes compressed gas from supply hose **49** to flow into the tubing roller **12** via the chamber **36** at a rate of about 2.5 to 3.5 ml per minute as determined by the setting of the first gas flow regulator **48**. This gas flow blows the separated supernatant from the tubing **21** back through the slip bearing **62**. Operation under these conditions continues for about 55 seconds which is sufficient to expel the supernatant, after which point the first gas valve **44** is closed.

Finally, the settled particles are dried by removing the residual evaporable liquid from the tubing **21**. To accomplish this, the programmable controller **52** increases the speed of rotation to between about 4000 and 6000 rpm, with 5000 rpm being preferred. This causes increased centrifugal forces sufficient to separate the particles from the evaporable liquid. The second valve **45** is thus actuated by the controller **52**, causing gas to flow into the tubing roller **12** at about 500 to 800 ml per minute as determined by the setting of the second gas flow regulator **48**. This increased gas flow evaporates the liquid from the suspension, leaving a uniform dispersion of the coated particles adhered to the concave arcuate inner surface of the tubing **21**. The final drying step lasts for approximately two minutes or until the particles are completely dry. After the drying cycle is finished, the programmable controller **52** closes the second gas valve **45** and directs the speed control **32** to stop the rotator means **26.** The coated tubing **21** then may be removed from the deposition apparatus **10**.

Before the tubing is cut into suitable lengths for use as sample cartridges, it is necessary to remove any end portions of the tubing in which particle distribution is uneven. The distribution of particles in the tubing can be tested operationally in using a gas driven particle acceleration apparatus under actual delivery conditions. The following test conditions are suitable, although other tests for determining and comparing the particle delivery profile of prepared sample cartridges can readily be devised.

Test cartridges of desired length are removed from opposite ends of the tubing. The particles from each test cartridge are delivered from the concave inner surface of the tubing under a gas pressure of around 400 psi, and are directed into minimal water (3%) agar in a 60 ml petri dish without surface condensation. From each plate, a slice approximately one cm long is cut through the center of the target agar and mounted onto a microscope slide. It is important to test slices of comparable thicknesses when samples are compared.

The slices are analyzed for particle delivery depth and particle number a microscope. The particles can be readily observed using a microscope having a 10X eyepiece equipped with a micrometer. At the top surface of the agar, the particles are most dense, with density decreasing along with the increasing depth of the agar slice. Areas of high, medium, and low particle density are noted in each slice. The eyepiece micrometer is aligned to zero at a depth approximately equal to the deepest penetration of the particles. The micrometer value at the agar surface is the particle depth. Typical particle depths after delivery into minimal water agar at 400 psi are about 100 to 120 µm when 0.95µ amorphous gold particles are used, and about 260 300 µm when 1 to 3µ gold spherical particles or beads are used.

If particle depth and density are similar, cartridges derived from the tubing section between the ends are acceptable for use. However, should the two ends differ markedly from each other, or standard coating parameters, additional pairs of opposite end samples should be tested until both ends yield comparable acceptable results. When comparable results are obtained from both ends, the remaining length of tubing is cut into pieces of suitable length using a scalpel and a ruler or other type of cutting device. The sample cartridges can then be stored at 4°C with desiccant in a Parafilm-sealed and labelled vial for up to two months.

Accordingly, a novel method and apparatus for depositing particles within a length of tubing have been described.

## Claims

1. An apparatus (10) for depositing particles within a length of tubing (21), the apparatus comprising:
a tubing roller (12) having an elongate tubing bore (20) formed therein, wherein said bore has first and second ends and is sized for removable insertion of a length of tubing (21) therein;
means (26) for rotating the tubing roller (12) about the major axis (27) of the tubing bore (20);
gas delivery means (24) comprising a chamber (36) with an inlet (38) for introducing gas from an associated source into the chamber, said chamber having an aperture through which a portion of the tubing roller (12) extends, wherein said aperture provides fluid communication between the second end of the tubing bore (20) and the chamber (36); and
support means (60) arranged adjacent to the first end of the tubing bore (20), wherein said support means provides for the sealable engagement between an end of a length of tubing (21) inserted into the tubing bore (20) and an associated source of particles to be deposited within the length of tubing.

2. The apparatus of claim 1 further comprising a bearing mount (19) for supporting the tubing roller (12) while allowing the rotational motion thereof.

3. The apparatus of claim 1 or claim 2, wherein the means (26) for rotating the tubing roller comprises a variable speed rotator.

4. The apparatus of claim 3, wherein the variable speed rotator comprises a motor driven by a variable speed control device (32).

5. The apparatus of any one of the preceding claims, further comprising a first valve (44) connected to the chamber inlet (38), wherein said first valve controls the passage of gas from the associated source into the chamber (36) via a first gas delivery path.

6. The apparatus of claim 5 further comprising a second valve (45) connected to the chamber inlet (38), wherein said second valve controls the passage of gas from the associated source into the chamber (36) via a second gas delivery path.

7. The apparatus of claim 5, further comprising a first gas flow regulator (48) which controls the flow of gas through the first valve (44).

8. The apparatus of claim 6 further comprising a first gas flow regulator (48) which controls the flow of gas through the first valve (44), and a second gas flow regulator (50) which controls the flow of gas through the second valve (45).

9. The apparatus of claim 8, wherein said first gas flow regulator (48) limits the gas flow through the first valve (44) at a first rate, and said second gas flow regulator (50) limits gas flow through the second valve (45) at a second rate.

10. The apparatus of any one of the preceding claims, further comprising a chamber outlet (38) which provides a path through which gas can exit from the chamber (36).

11. The apparatus of claim 10 further comprising a third valve (46) connected to the chamber outlet (38), wherein the third valve controls the passage of gas from the chamber (36) to an exhaust means.

12. The apparatus of claim 11 when dependent from claim 6, wherein the first, second and third valves (44,45,46) are solenoid valves.

13. The apparatus of claim 12 when dependent from claim 4, wherein the variable speed control device (32) and the first, second and third valves *(*44,45,46) are operably connected with a programmable controller (52) which controls actuation of said valves and said variable speed control device, thereby providing for the automated operation of said apparatus.

14. The apparatus of claim 13, further comprising a microprocessor for controlling the operation of the programmable controller (52).

15. A method for depositing particles in a length of tubing (21) having a longitudinal axis (27) and a curved interior surface, the method comprising the steps of:
(a) preparing a uniformly dispersed suspension of particles coated with a biological substance in an evaporable liquid;
(b) rotating the tubing (21) about its longitudinal axis (27) at a first speed;
(c) introducing the particle suspension into the tubing while rotating said tubing at the first speed;
(d) rotating the tubing to centrifugally separate the particles from the evaporable liquid and distribute the particles on the interior surface of the tubing; and
(e) passing a gas through the tubing as the tubing rotates to dry the particles distributed on the interior surface.

16. The method of claim 15, wherein step (c) comprises: loading a syringe (64) with the suspension; coupling the syringe to an end of the tubing (21); and transferring the suspension from the syringe into the tubing.

17. The method of claim 15 or claim 16, wherein steps (b) through (e) are performed with the longitudinal axis (27) of the tubing (21) oriented horizontally.

18. The method of any one of claims 15 to 17, wherein the tubing (21) is rotated at a speed of about 50 to 200 revolutions per minute in step (b).

19. The method of any one of claims 15 to 18, wherein the tubing is rotated in step (d) at a second speed which is greater than the first speed.

20. The method of any one of claims 15 to 19, wherein the second speed is between about 1700 and 2300 revolutions per minute.

21. The method of any one of claims 15 to 20, wherein the tubing (21) is rotated in step (e) at a speed which is greater than the first speed.

22. The method of any one of claims 15 to 21, wherein step (e) comprises:
passing a gas at a first flow rate through the tubing (21) at the tubing rotates to remove the evaporable liquid therefrom; and
passing a gas at a second flow rate through the tubing as the tubing rotates to dry the particles distributed on the interior surface of said tubing, wherein the second flow rate is greater than the first flow rate.

23. The method of claim 22, wherein the step of passing a gas at a first flow rate occurs while the tubing (21) is rotating at a third speed which is less than the second speed.

24. The method of claim 23, wherein the third speed is between about 700 and 1300 revolutions per minute.

25. The method of claim 23 or claim 24, wherein the step of passing a gas at a second flow rate occurs while the tubing is rotating at a fourth speed which is greater than the third speed.

26. The method of claim 25, wherein the fourth speed is between about 4000 and 6000 revolutions per minute.

27. The method of any one of claims 22 to 26, wherein the first flow rate is between about 2.5 and 3.5 milliliters per minute and/or the second flow rate is between about 500 and 800 milliliters per minute.

28. The method of any one of claims 15 to 27, wherein the gas is selected from the group consisting of air and nitrogen.

## Patentansprüche

1. Apparat (10) zur Ablagerung von Partikeln innerhalb der Röhrenlänge (21), welcher Apparat umfasst:
einen Röhrenwalze (12) mit einer darin erzeugten langen Röhrenbohrung (20), wobei diese Bohrung ein erstes und ein zweites Ende aufweist und zur wieder entnehmbaren Einführung einer Röhrenlänge (21) in sie hinein dimensioniert ist.
Vorrichtung (26) zur Rotation der Röhrenwalze (12) um die Hauptachse (27) der Röhrenbohrung (20);
Gaszufuhrvorrichtung (24), umfassend eine Kammer (36) mit einem Einlass (38) zur Einführung von Gas von einer damit verbundenen Quelle in die Kammer, welche Kammer eine Öffnung aufweist, durch die sich ein Teil der Röhrenwalze (12) erstreckt, wobei die Öffnung eine Flüssigkeitsverbindung zwischen dem zweiten Ende der Röhrenbohrung (20) und der Kammer (36) schafft; und
Trägervorrichtung (60), die angrenzend an das erste Ende der Röhrenbohrung (20) angeordnet ist, wobei die Trägervorrichtung eine verschließbare Kupplung zwischen einem Ende der Röhrenlänge (21), die in die Röhrenbohrung (20) eingeführt ist, und einer damit verbundenen Quelle von Partikeln, welche innerhalb der Röhrenlänge abzulagern sind, ermöglicht.

2. Apparat nach Anspruch 1, außerdem umfassend eine Lageraufsatz (19) zum Tragen der Röhrenwalze (12) bei gleichzeitiger Ermöglichung ihrer Rotationsbewegung.

3. Apparat nach Anspruch 1 oder Anspruch 2, wobei die Vorrichtung (26) zur Rotation der Röhrenwalze einen Rotor mit veränderlicher Geschwindigkeit umfasst.

4. Apparat nach Anspruch 3, wobei der Rotor mit veränderlicher Geschwindigkeit einen Motor umfasst, der durch eine Kontrollvorrichtung mit veränderlicher Geschwindigkeit (32) angetrieben wird.

5. Apparat nach einem der vorangehenden Ansprüche, außerdem umfassend ein erstes Ventil (44), das an den Kammereinlass (38) angeschlossen ist, wobei das erste Ventil den Gasdurchgang von der damit verbundenen Quelle in die Kammer (36) über einen ersten Gaszufuhrweg kontrolliert.

6. Apparat nach Anspruch 5, außerdem umfassend ein zweites Ventil (45), das an den Kammereinlass (38) angeschlossen ist, wobei das zweite Ventil den Gasdurchgang von der damit verbundenen Quelle in die Kammer (36) über einen zweiten Gaszufuhrweg kontrolliert.

7. Apparat nach Anspruch 5, außerdem umfassend einen ersten Gasstromregulator (48), der den Gasstrom durch das erste Ventil (44) kontrolliert.

8. Apparat nach Anspruch 6, außerdem umfassend einen ersten Gasstromregulator (48), welcher den Gasstrom durch das erste Ventil (44) kontrolliert, und einen zweiten Gasstromregulator (50), welcher den Gasstrom durch das zweite Ventil (45) reguliert.

9. Apparat nach Anspruch 8, wobei der erste Gasstromregulator (48) den Gasstrom durch das erste Ventil (44) bei einer ersten Geschwindigkeit begrenzt, und der zweite Gasstromregulator (50) den Gasstrom durch das zweite Ventil (45) bei einer zweiten Geschwindigkeit begrenzt.

10. Apparat nach einem der vorangehenden Ansprüche, außerdem umfassend einen Kammerauslass (38), der einen Weg bereitstellt, durch den Gas aus der Kammer (36) austreten kann.

11. Apparat nach Anspruch 10, außerdem umfassend ein drittes Ventil (46), das an den Kammerauslass (38) angeschlossen ist, wobei das dritte Ventil den Gasdurchgang aus der Kammer (36) zu einer Abgasvorrichtung kontrolliert.

12. Apparat nach Anspruch 11, wenn abhängig von Anspruch 6, wobei die ersten, zweiten und dritten Ventile (44,45,46) elektromagnetische Ventile sind.

13. Apparat nach Anspruch 12, wenn abhängig von Anspruch 4, wobei die Kontrollvorrichtung mit veränderlicher Geschwindigkeit (32) und die ersten, zweiten und dritten Ventile (44,45,46) funktionsfähig an eine programmierbare Steuereinheit (52) angeschlossen sind, welche die Betätigung der Ventile und der Kontrollvorrichtung mit veränderlicher Geschwindigkeit kontrolliert, wodurch der automatisierte Betrieb des Apparats ermöglicht wird.

14. Apparat nach Anspruch 13, außerdem umfassend einen Mikroprozessor zur Kontrolle des Betriebs der programmierbaren Steuereinheit (52).

15. Verfahren zur Ablagerung von Partikeln in einer Röhrenlänge (21) mit einer Längsachse (27) und einer gebogenen inneren Oberfläche, welches Verfahren die folgenden Schritte umfasst:
(a) Herstellen einer gleichmäßig dispergierten Suspension von Partikeln, die mit einer biologischen Substanz beschichtet sind, in einer verdampfbaren Flüssigkeit;
(b) Rotieren der Röhre (21) um ihre Längsachse (27) bei einer ersten Geschwindigkeit;
(c) Einführen der Partikelsuspension in die Röhre bei gleichzeitiger Rotation der Röhre bei der ersten Geschwindigkeit;
(d) Rotieren der Röhre zur zentrifugalen Abtrennung der Partikel von der verdampfbaren Flüssigkeit und Verteilen der Partikel auf der inneren Oberfläche der Röhre; und
(e) Führen eines Gases durch die Röhre während der Rotation der Röhre, um die auf der inneren Oberfläche verteilten Partikel zur trocknen.

16. Verfahren nach Anspruch 15, wobei Schritt (c) umfasst: Aufziehen einer Spritze (64) mit der Suspension; Kuppeln der Spritze an ein Ende der Röhre (21); und Übertragen der Suspension von der Spritze in die Röhre.

17. Verfahren nach Anspruch 15 oder Anspruch 16, wobei die Schritte (b) bis (e) bei einer horizontal orientierten Längsachse (27) der Röhre (21) durchgeführt werden.

18. Verfahren nach einem der Ansprüche 15 bis 17, wobei die Röhre (21) bei einer Geschwindigkeit von etwa 50 bis 200 Umdrehungen pro Minute bei Schritt (b) rotiert wird.

19. Verfahren nach einem der Ansprüche 15 bis 18, wobei die Röhre bei Schritt (d) bei einer zweiten Geschwindigkeit rotiert wird, die größer als die erste Geschwindigkeit ist.

20. Verfahren nach einem der Ansprüche 15 bis 19, wobei die zweite Geschwindigkeit zwischen etwa 1700 und 2300 Umdrehungen pro Minute liegt.

21. Verfahren nach einem der Ansprüche 15 bis 20, wobei die Röhre (21) bei Schritt (e) bei einer Geschwindigkeit rotiert wird, die größer als die erste Geschwindigkeit ist.

22. Verfahren nach einem der Ansprüche 15 bis 21, wobei Schritt (e) umfasst:
Durchführen eines Gases bei einer ersten Stromgeschwindigkeit durch die Röhre (21), während die Röhre rotiert wird, um die verdampfbare Flüssigkeit davon zu entfernen;
Durchführen eines Gases bei einer zweiten Stromgeschwindigkeit durch die Röhre, während die Röhre rotiert wird, um die auf der inneren Oberfläche der Röhre verteilten Partikel zu trocknen, wobei die zweite Stromgeschwindigkeit größer als die erste Stromgeschwindigkeit ist.

23. Verfahren nach Anspruch 22, wobei der Schritt des Durchführens eines Gases bei einer ersten Stromgeschwindigkeit erfolgt, während die Röhre (21) bei einer dritten Geschwindigkeit rotiert wird, die geringer als die zweite Geschwindigkeit ist.

24. Verfahren nach Anspruch 23, wobei die dritte Geschwindigkeit zwischen etwa 700 und 1300 Umdrehungen pro Minute beträgt.

25. Verfahren nach Anspruch 23 oder Anspruch 24, wobei der Schritt des Durchführens eines Gases bei einer zweiten Stromgeschwindigkeit erfolgt, während die Röhre bei einer vierten Geschwindigkeit rotiert wird, die größer als die dritte Geschwindigkeit ist.

26. Verfahren nach Anspruch 25, wobei die vierte Geschwindigkeit zwischen etwa 4000 und 6000 Umdrehungen pro Minute beträgt.

27. Verfahren nach einem der Ansprüche 22 bis 26, wobei die erste Stromgeschwindigkeit zwischen etwa 2,5 und 3,5 Milliliter pro Minute und/oder die zweite Stromgeschwindigkeit zwischen etwa 500 und 800 Milliliter pro Minute beträgt.

28. Verfahren nach einem der Ansprüche 15 bis 27, wobei das Gas gewählt ist aus der Gruppe, bestehend aus Luft und Stickstoff.

## Revendications

1. Dispositif (10) pour déposer des particules dans une longueur de tube (21), le dispositif comportant :
un rouleau pour tube (12) ayant un alésage pour tube allongé (20) formé à l'intérieur, ledit alésage ayant des première et seconde extrémités et étant dimensionné pour une insertion amovible d'une longueur de tube (21) à l'intérieur de celui-ci,
des moyens (26) pour faire tourner le rouleau pour tube (12) autour de l'axe principal (27) de l'alésage pour tube (20),
des moyens de distribution de gaz (24) comportant une chambre (36) ayant une entrée (38) pour introduire un gaz depuis une source associée dans la chambre, ladite chambre ayant une ouverture à travers laquelle s'étend une partie du rouleau pour tube (12), ladite ouverture établissant une communication de fluide entre la seconde extrémité de l'alésage pour tube (20) et la chambre (36), et
des moyens de support (60) agencés à proximité adjacente de la première extrémité de l'alésage pour tube (20), lesdits moyens de support assurant un engagement pouvant être rendu étanche entre une extrémité d'une longueur de tube (21) insérée dans l'alésage pour tube (20) et une source associée de particules à déposer dans la longueur de tube.

2. Dispositif selon la revendication 1, comportant de plus un montage de palier (19) pour supporter le rouleau pour tube (12) tout en permettant le mouvement rotationnel de celui-ci.

3. Dispositif selon la revendication 1 ou 2, dans lequel les moyens (26) pour faire tourner le rouleau pour tube comportent un dispositif de mise en rotation à vitesse variable.

4. Dispositif selon la revendication 3, dans lequel le dispositif de mise en rotation à vitesse variable comporte un moteur entraîné *par* un dispositif de commande de vitesse variable (32).

5. Dispositif selon l'une quelconque des revendications précédentes, comportant une première vanne (44) connectée à l'entrée de chambre (38), ladite première vanne commandant le passage de gaz depuis la source associée dans la chambre (36) via un premier trajet de distribution de gaz.

6. Dispositif selon la revendication 5, comportant de plus une deuxième vanna (45) connectée à l'entrée de chambre (38), ladite deuxième vanne commandant le passage de gaz depuis la source associée dans la chambre (36) via un second trajet de distribution de gaz.

7. Dispositif selon la revendication 5, comportant de plus un premier régulateur d'écoulement de gaz (48) qui commande l'écoulement de gaz à travers la première vanne (44).

8. Dispositif selon la revendication 6, comportant de plus un premier régulateur d'écoulement de gaz (48) qui commande l'écoulement de gaz à travers la première vanne (44), et un second régulateur d'écoulement de gaz (50) qui commande l'écoulement de gaz à travers la deuxième vanne (45) .

9. Dispositif selon la revendication 8, dans lequel le premier régulateur d'écoulement de gaz (48) limite l'écoulement de gaz à travers la première vanne (44) à une première vitesse, et ledit second régulateur d'écoulement de gaz (50) limite l'écoulement de gaz à travers la deuxième vanne (45) à une deuxième vitesse.

10. Dispositif selon l'une quelconque des revendications précédentes, comportant de plus une sortie de chambre (38) qui établit un trajet à travers lequel un gaz peut sortir depuis la chambre (36).

11. Dispositif selon la revendication 10, comportant de plus une troisième vanne (46) connectée à la sortie de chambre (38), la troisième vanne commandant le passage de gaz depuis la chambre (36) vers des moyens d'échappement.

12. Dispositif selon la revendication 11, lorsqu'elle est dépendante de la revendication 6, dans lequel les première, deuxième et troisième vannes (44, 45, 46) sont des électrovannes.

13. Dispositif selon la revendication 12 lorsqu'elle est dépendante de la revendication 4, dans lequel le dispositif de commande de vitesse variable (32) et les première, deuxième et troisième vannes (44, 45, 46) sont connectés de manière opérationnelle à un contrôleur programmable (52) qui commande 1'actionnement desdites vannes et dudit dispositif de commande de vitesse variable, de manière à permettre un fonctionnement automatisé dudit dispositif.

14. Dispositif selon la revendication 13, comportant de plus un microprocesseur pour commander le fonctionnement du contrôleur programmable (52).

15. Procédé pour déposer des particules dans une longueur de tube (21) ayant un axe longitudinal (27) et une surface intérieure incurvée, le procédé comportant les étapes consistant à :
(a) préparer une suspension de particules uniformément dispersées revêtues d'une substance biologique dans un liquide pouvant être évaporé,
(b) mettre en rotation le tube (21) autour de son axe longitudinal (27) à une première vitesse,
(c) introduire la suspension de particules dans le tube tout en mettant en rotation ledit tube à la première vitesse,
(d) mettre en rotation le tube pour séparer par centrifugation les particules du liquide pouvant être évaporé et répartir les particules sur la surface intérieure du tube, et
(e) faire passer un gaz à travers le tube lorsque le tube tourne pour sécher les particules réparties sur la surface intérieure.

16. Procédé selon la revendication 15, dans lequel l'étape (c) comporte : le remplissage d'une seringue (64) à l'aide de la suspension, le couplage de la seringue à l'extrémité du tube (21), et le transfert de la suspension depuis la seringue dans le tube.

17. Procédé selon la revendication 15 ou 16, dans lequel les étapes (b) à (e) sont effectuées en ayant l'axe longitudinal (27) du tube (21) orienté horizontalement.

18. Procédé selon l'une quelconque des revendications 15 à 17, dans lequel le tube (21) est mis en rotation à une vitesse d'environ 50 à 200 tours par minute à 1' étape (b) .

19. Procédé selon 1'une quelconque des revendications 15 à 18, dans lequel le tube est mis en rotation à l'étape (d) à une deuxième vitesse qui est supérieure à la première vitesse.

20. Procédé selon l'une quelconque des revendications 15 à 19, dans lequel la deuxième vitesse est comprise entre environ 1700 et 2300 tours par minute.

21. Procédé selon l'une quelconque des revendications 15 à 20, dans lequel le tube (21) est mis en rotation à l'étape (e) à une vitesse qui est supérieure à la première vitesse.

22. Procédé selon l'une quelconque des revendications 15 à 21, dans lequel l'étape (e) comporte :
le passage d'un gaz à un premier débit à travers le tube (21) lorsque le tube tourne pour éliminer le liquide pouvant être évaporé depuis celui-ci, et
le passage d'un gaz à un second débit, à travers le tube lorsque le tube tourne pour sécher les particules réparties sur la surface intérieure dudit tube, le second débit étant supérieur au premier débit.

23. Procédé selon la revendication 22, dans lequel l'étape de passage d'un gaz à un premier débit a lieu alors que le tube (21) est en rotation à une troisième vitesse qui est inférieure à la deuxième vitesse.

24. Procédé selon la revendication 23, dans lequel la troisième vitesse est comprise entre 700 et 1300 tours par minute.

25. Procédé selon la revendication 23 ou 24, dans lequel l'étape de passage d'un gaz à un second débit a lieu alors que le tube est mis en rotation à une quatrième vitesse qui est supérieure à la troisième vitesse.

26. Procédé selon la revendication 25, dans lequel la quatrième vitesse est comprise entre environ 4000 et 6000 tours par minute.

27. Procédé selon l'une quelconque des revendications 22 à 26, dans lequel le premier débit est compris entre environ 2,5 et 3,5 millimètres par minute et/ou le second débit est compris entre environ 500 et 800 millimètres par minute.

28. Procédé selon l'une quelconque des revendications 15 à 27, dans lequel le gaz est sélectionné parmi le groupe constitué d'air et d'azote.
